(19) Europäisches Patentamt
European Patent Office
Office européen des brevets

(11) Numéro de publication : **0 097 071**
**B1**

(12) # FASCICULE DE BREVET EUROPÉEN

(45) Date de publication du fascicule du brevet : **28.08.85**

(21) Numéro de dépôt : **83401046.4**

(22) Date de dépôt : **26.05.83**

(51) Int. Cl.[4] : **C 07 C147/14**, C 07 D295/18, A 61 K 31/16, A 61 K 31/535, A 61 K 31/445// C07C149/22, C07C149/23, C07C33/24

(54) **Dérivés de benzhydrylsulfinylacétamide et leur utilisation en thérapeutique.**

(30) Priorité : **04.06.82 FR 8209802**

(43) Date de publication de la demande : **28.12.83 Bulletin 83/52**

(45) Mention de la délivrance du brevet : **28.08.85 Bulletin 85/35**

(84) Etats contractants désignés : **AT BE CH DE FR GB IT LI LU NL SE**

(56) Documents cités : **FR-A- 2 385 693**

(73) Titulaire : **LABORATOIRE L. LAFON Société anonyme dite: 1 rue Georges Médéric F-94701 Maisons-Alfort (FR)**

(72) Inventeur : **Lafon, Louis 5 rue de l'Alboni F-75016 Paris (FR)**

(74) Mandataire : **Combe, André et al CABINET BEAU DE LOMENIE 55 rue d'Amsterdam F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 097 071 B1

**Description**

La présente invention a trait à de nouveaux dérivés de benzhydrylsulfinylacétamide en tant que produits industriels nouveaux. Elle a trait également à l'utilisation de ces nouveaux dérivés en thérapeutique, notamment en tant que médicaments actifs sur le système nerveux central (SNC).

On sait selon FR-A-2 385 693 que le benzhydrylsulfinylacétamide (décrit à l'exemple 1 et ayant pour numéro de code « CRL 40476 ») présente un spectre neuropsychopharmacologique particulier :

— présence :
d'excitation avec hyperréactivité,
d'hypermotilité, et

— absence :
de stéréotypies (sauf à fortes doses),
de potentialisation des effets de l'apomorphine et de l'amphétamine.

On préconise à présent de nouveaux dérivés de benzhydrylsulfinylacétamide qui ont des propriétés pharmacologiques intéressantes.

Les nouveaux dérivés de benzhydrylsulfinylacétamide selon l'invention sont caractérisés en ce qu'ils répondent à la formule générale

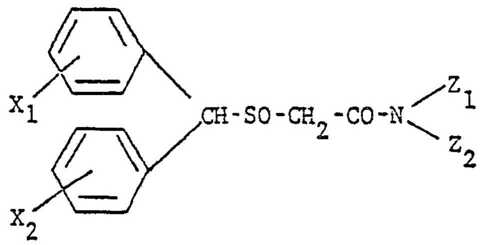

(I)

dans laquelle

$X_1$ et $X_2$, qui peuvent être identiques ou différents, représentent chacun H, Cl ou F,

$Z_1$ représente $CH_3$, $CH_2CH_3$, $CH(CH_3)_2$ ou $C(CH_3)_3$, $Z_1$ pouvant représenter en outre un atome d'hydrogène quand au moins un des $X_1$ et $X_2$ est différent de H,

$Z_2$ représente H ; $NZ_1Z_2$ considérés ensemble pouvant représenter un groupe pipéridino ou morpholino.

Les groupes $X_1$ et $X_2$ préférés sont H, 4-Cl et 4-F.

Dans le tableau I ci-après, on a consigné un certain nombre de composés selon la formule I de façon nullement limitative, en mentionnant l'activité essentielle sur le SNC.

Les produits de formule I peuvent être préparés selon une méthode connue en soi par application de mécanismes réactionnels classiques, par exemple en mettant en œuvre une des deux méthodes décrites dans FR-A-2 385 693. Le procédé que l'on préconise ici consiste :

1. à faire réagir un chlorure d'acide de formule

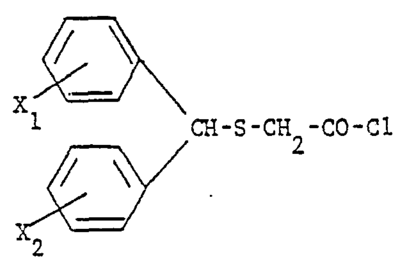

(II)

(où $X_1$ et $X_2$ sont définis comme ci-dessus) avec une amine

$$HNZ_1Z_2 \qquad (III)$$

(où $Z_1$ et $Z_2$ sont définis comme ci-dessus) pour obtenir un dérivé de benzhydrylthioacétamide de formule

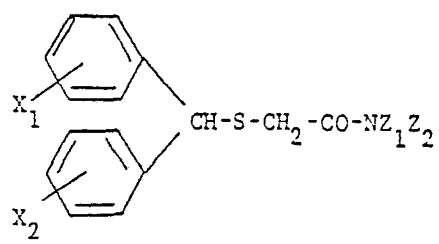

(IV)

(où $X_1$, $X_2$, $Z_1$ et $Z_2$ sont définis comme ci-dessus), et

2. à soumettre ledit dérivé de benzhydrylthioacétamide à une réaction d'oxydation au moyen de $H_2O_2$ à 110-130 volumes à une température inférieure ou égale à 45 °C.

Les composés de formule I agissent tous sur le SNC. Les produits des exemples 1-3 et 7-8 sont des agents stimulants, en revanche les produits des exemples 4-6 sont des agents sédatifs.

On préconise une composition thérapeutique qui est caractérisée en ce qu'elle renferme en association avec un excipient physiologiquement acceptable au moins un composé de formule I en tant qu'ingrédient actif. Cet ingrédient actif est utilisé bien entendu à une teneur pharmaceutiquement efficace.

(Voir tableau I, page 4)

Tableau I

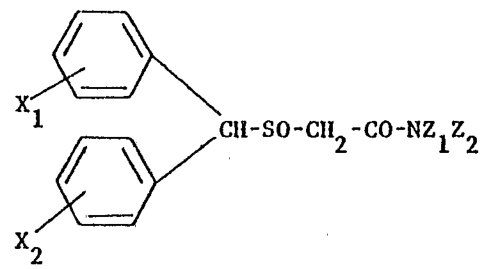

| Produit | n° de code | $X_1$ | $X_2$ | $NZ_1Z_2$ | Point de fusion | Activité sur le SNC |
|---|---|---|---|---|---|---|
| Exemple 1 | CRL 40920 | H | H | $NHCH_3$ | 80-85°C | stimulant |
| Exemple 2 | CRL 40929 | H | H | $NHCH(CH_3)_2$ | 120°C | stimulant |
| Exemple 3 | CRL 40929A | H | H | $NHC(CH_3)_3$ | - | stimulant |
| Exemple 4 | CRL 40936 | H | H | $NHCH_2CH_3$ | 90°C | sédatif |
| Exemple 5 | CRL 40489 | H | H | pipéridino | 150°C | sédatif |
| Exemple 6 | CRL 40489A | H | H | morpholino | - | sédatif |
| Exemple 7 | CRL 40933 | 4-Cl | H | $NH_2$ | 145-146°C | stimulant |
| Exemple 8 | CRL 40940 | 4-F | 4-F | $NH_2$ | 101-102°C | stimulant |
| Exemple 9 | CRL 41018 | 4-F | H | $NH_2$ | 159°C | stimulant |
| Produit comparatif | CRL 40476 | H | H | $NH_2$ | 164-166°C | stimulant |

Les composés selon l'invention se distinguent du benzhydrylsulfinylacétamide (CRL 40476) précité par une différence d'action vis-à-vis du sommeil barbiturique. Plus précisément, les composés selon l'invention diminuent nettement le sommeil barbiturique induit (lot de 10 animaux par dose et par produit à tester) chez la souris mâle par administration de pentobarbital (50 mg/kg i. p.) 0,5 h après administration i. p. de produit à tester, alors que le CRL 40476 ne modifie pas la durée du sommeil barbiturique induit par le pentobarbital. Cette différence d'action qui est intéressante sur le plan thérapeutique est illustrée par le tableau IV

Tableau IV

| Produit | n° de code | Action sur la durée du sommeil induit par le pentobarbital (50 mg/kg i.p.) | |
|---|---|---|---|
| | | dose | effet |
| exemple 1 | CRL 40920 | 128 mg/kg i.p. | (a) |
| exemple 2 | CRL 40929 | 256 mg/kg i.p. | (a) |
| exemple 4 | CRL 40936 | 64 mg/kg i.p. | (a) |
| exemple 4 | CRL 40936 | 128 mg/kg i.p. | (a) |
| exemple 4 | CRL 40936 | 256 mg/kg i.p. | (a) |
| exemple 5 | CRL 40489 | 128 mg/kg i.p. | (a) |
| exemple 7 | CRL 40933 | 8 mg/kg i.p. | (a) |
| exemple 8 | CRL 40940 | 32 mg/kg i.p. | (a) |
| exemple 9 | CRL 41018 | 16 mg/kg i.p. | (a) |
| produit de comparaison | CRL 40476 | de 8 à 256 mg/kg i.p. | (b) |

Notes :

(a) diminution nette de la durée du sommeil.
(b) pas de modification significative de la durée du sommeil.

On a donné ci-après quelques exemples de préparation nullement limitatifs mais donnés à titre d'illustration de composés selon l'invention.

Préparation I

Obtention du N-méthyl-benzhydrylsulfinylacétamide.
(Exemple 1 ; n° de code : CRL 40920)

1) Acide benzhydrylthioacétique.

Dans un tricol de 2 l, on place 91,2 g (1,2 mole) de thiourée dans un mélange de 520 ml d'HBr à 48 % (p/v) et de 80 ml d'eau. On porte le milieu réactionnel à 60 °C et ajoute, en une seule fois, 184 g (1 mole) de benzhydrol. On élève la température jusqu'à 95 °C puis refroidit : le bromhydrate de thiouronium précipite sous forme de petites billes orangées. Celles-ci sont filtrées et réempâtées plusieurs fois dans l'eau.

Le sel de thiouronium ainsi obtenu est introduit dans un tricol avec 320 ml de lessive de soude (d = 1,38 ; 3,2 moles) ; on porte à 70 °C et ajoute goutte à goutte une solution de 103,95 g (1,1 mole) d'acide chloracétique dans 200 ml d'eau. A la fin de l'addition, on porte à reflux et maintient celui-ci pendant 30 min ; un insoluble se forme, celui-ci est écarté par filtration à chaud, puis le filtrat est refroidi et acidifié avec HCl concentré : l'acide benzhydrylthioacétique précipite. On essore celui-ci et lave à l'eau.

On purifiera l'acide benzhydrylthioacétique en le redissolvant à chaud dans une solution de soude

diluée ; la solution alcaline ainsi obtenue est filtrée sur charbon, refroidie et acidifiée avec HCl concentré. On essore le précipité ainsi obtenu et lave à l'eau.

On recueille 90,4 g (rendement = 35 %) d'acide benzhydrylthioacétique ($F._{inst.}$ = 130 °C).

2) Chlorure de l'acide benzhydrylthioacétique.

Dans un tricol, on place 25,8 g (0,1 mole) d'acide benzhydrylthioacétique dans 150 ml de benzène, chauffe le mélange et ajoute goutte à goutte à reflux 25 ml (0,342 mole) de chlorure de thionyle. Une fois l'addition terminée, on poursuit le reflux pendant environ 1 h-1,5 h, refroidit, évapore le benzène et le chlorure de thionyle en excès. On recueille ainsi une huile orangée limpide.

3) N-méthyl-benzhydrylthioacétamide.

Dans un tricol muni d'un réfrigérant, d'un thermomètre et d'une ampoule de coulée, on introduit 28 ml (environ 0,3 mole) de méthylamine dans 50 ml d'eau et ajoute goutte à goutte le chlorure d'acide précédemment obtenu (environ 0,1 mole) dissous dans environ 100 ml de chlorure de méthylène.

On laisse le mélange en contact une nuit ; le lendemain, on lave la phase organique à l'eau, puis avec une solution de soude diluée et de nouveau à l'eau. On sèche sur $Na_2SO_4$, évapore le chlorure de méthylène, reprend le précipité ainsi obtenu à l'éther diisopropylique.

On obtient après recristallisation dans l'isopropanol 22,3 g (rendement = 82 %) de N-méthyl-benzhydrylthioacétamide, ($F._{inst.}$ = 101-102 °C) qui se présente sous la forme d'une poudre blanche.

4) CRL 40920

On met dans un ballon 16,26 g (0,06 mole) de l'acétamide précédemment obtenu, ajoute 60 ml d'acide acétique et 6,6 ml d'$H_2O_2$ (à environ 130 volumes), la température monte jusqu'à 40 °C puis redescend. Lorsque la totalité de sulfure a disparu, on évapore l'acide acétique, reprend à l'eau : le N-méthyl-benzhydrylsulfinylacétamide précipite. On essore celui-ci et lave plusieurs fois à l'eau.

On recueille après recristallisation dans l'acétate d'éthyle 13,8 g (rendement = 80 %) de CRL 40920 (F. = 80-85 °C).

Le CRL 40920 est une poudre blanche, insoluble dans l'éther et l'acétate d'éthyle, soluble dans le méthanol, l'éthanol et l'acétone. Sa solubilité dans l'eau est inférieure à 1 g/l.

Préparation II

Obtention de la N-(benzhydrylsulfinylacétyl)-pipéridine.
(Exemple 5 ; n° de code : CRL 40489)

1) N-(benzhydrylthioacétyl)-pipéridine.

Dans un tricol muni d'un réfrigérant et d'une ampoule de coulée, on place 29 ml (0,3 mole) de pipéridine dans environ 50 ml d'éther et l'on ajoute goutte à goutte 27,2 g (0,098 mole) du chlorure de l'acide benzhydrylthioacétique en solution dans environ 100 ml de benzène. Une fois l'addition terminée, le mélange réactionnel est additionné d'eau. On lave la phase organique avec une solution de soude diluée, d'HCl dilué, puis à l'eau, sèche sur $Na_2SO_4$, évapore le solvant, reprend avec un mélange éther diisopropylique-éther de pétrole ; on cristallise ainsi le produit attendu. On recueille après recristallisation dans l'acétate d'éthyle 21,2 g (rendement = 68 %) de N-(benzhydrylthioacétyl)-pipéridine. ($F._{inst.}$ = 82-83 °C).

2) CRL 40489

On met dans un ballon 16,25 g (0,05 mole) de N-(benzhydrylthioacétyl)-pipéridine et ajoute 50 ml d'acide acétique et 5,3 ml de $H_2O_2$ (à environ 110 volumes). On laisse le milieu réactionnel en contact une nuit à 15-25 °C, puis évapore l'acide acétique. On reprend le résidu d'évaporation avec de l'éther pour cristalliser le produit attendu. Par recristallisation dans l'acétate d'éthyle, on obtient 12,7 g (rendement = 77 %) de CRL 40489 ($F._{inst.}$ = 150 °C). La solubilité de ce produit dans l'eau est inférieure à 1 g/l.

Préparation III

Obtention du 4-chlorobenzhydrylsulfinylacétamide.

(Exemple 7 ; n° de code : CRL 40933).

1) 4-chlorobenzhydrol.

Dans une solution de 0,35 mole de bromure de phénylmagnésium dans 300 ml d'éther anhydre, on coule à 20 °C une solution de 49,2 g (0,35 mole) de parachlorobenzaldéhyde dans 300 ml d'éther anhydre. Après 2 h de reflux et une nuit au repos, on essore le précipité, lave avec 2 fois 50 ml d'éther et jette le filtrat résultant dans un mélange violemment agité de 200 ml d'HCl 6N et 500 g de glace. On essore le précipité, lave à l'eau, sèche et recristallise dans le mélange méthanol-eau (80 : 20) v/v ; on obtient le 4-chlorobenzhydrol (F = 60-61 °C) avec un rendement de 70 %.

2) Acide 4-chlorobenzhydrylthioacétique.

7,6 g (0,1 mole) de thiourée sont mis en solution dans 50 ml d'HBr à 48 %. On chauffe à 40 °C et ajoute simultanément 50 ml d'éthanol et 17,5 g (0,08 mole) de 4-chlorobenzhydrol, puis chauffe 0,25 h à reflux. On évapore l'alcool sous vide, ajoute 50 ml d'eau froide, essore le sel de thiouronium et lave avec 2 fois 20 ml d'eau froide. Le précipité humide est mis en suspension dans 100 ml d'eau, on ajoute une solution de 16 g de soude dans 100 ml d'eau et chauffe 0,25 h au bain-marie bouillant. On ajoute ensuite goutte à goutte à 60-70 °C une solution de 9,45 g (0,1 mole) d'acide chloroacétique, 5 g de $CO_3Na_2$ et 100 ml d'eau. On chauffe 1 h au bain-marie bouillant, acidifie avec HCl 3N, extrait à l'éther, extrait la phase éthéré, au bicarbonate dilué, filtre la solution alcaline sur charbon, précipite avec HCl 3N, essore, lave à l'eau, sèche et obtient l'acide attendu (F = 101-102 °C) avec un rendement de 90 %.

3) 4-chlorobenzhydrylthioacétamide.

On chauffe 1 h à reflux une solution de 11,7 g (0,04 mole) d'acide 4-chlorobenzhydrylthioacétique dans 60 ml de benzène et 12 ml de $SOCl_2$, évapore sous vide, reprend l'huile avec 50 ml d'éther et verse lentement, à froid en agitant dans 50 ml d'ammoniaque à 28 %. On agite 1 h, décante l'éther, lave à l'eau, sèche, chasse l'éther sous vide, cristallise le résidu dans l'éther de pétrole et obtient l'amide attendu (F = 52-53 °C) avec un rendement de 90 %.

4) CRL 40933.

12,3 g (0,042 mole) de 4-chlorobenzhydrylthioacétamide en solution dans 40 ml d'acide acétique sont oxydés à 40-45 °C avec 4,2 ml d'eau oxygénée à 110 volumes. L'acide acétique est évaporé sous vide, le résidu est cristallisé dans l'éther diisopropylique et recristallisé dans l'acétate d'éthyle. On obtient ainsi le CRL 40933 avec un rendement de 52 %. F = 145-146 °C.

## Préparation IV

Obtention du 4,4'-difluorobenzhydrylsulfinylacétamide.
(Exemple 8 ; n° de code : CRL 40940)

1) Acide 4,4'-difluorobenzhydrylthioacétique.

Dans une solution à 50 °C de 11,4 g (0,15 mole) de thiourée dans 75 ml d'HBr à 48 %, on ajoute simultanément 75 ml d'éthanol et 26,4 g (0,12 mole) de 4,4'-difluorobenzhydrol. On chauffe 0,25 h à reflux, évapore l'alcool sous vide, ajoute 50 ml d'eau froide, essore et lave avec deux fois 20 ml d'eau froide. On recueille le précipité, puis on le met en suspension dans 150 ml d'eau. On traite le mélange résultant avec une solution de 20 g (0,5 mole) de soude dans 100 ml d'eau. On porte 0,25 h à reflux, puis ajoute à 70-75 °C, goutte à goutte une solution de 18 g d'acide chloroacétique, 100 ml d'eau et 9 g de carbonate de sodium. Après 1 h à reflux, on acidifie avec HCl 3N, essore, lave à l'eau. On reprend à l'eau, traite au bicarbonate de sodium, filtre sur charbon et précipite avec HCl 3N. Après essorage, lavage à l'eau puis séchage, on obtient l'acide attendu (F. = 117-118 °C) avec un rendement de 87 %.

2) 4,4'-difluorobenzhydrylthioacétamide.

On chauffe 1 h à reflux une solution de 14,7 g (0,05 mole) d'acide 4,4'-difluorobenzhydrylthioacétique dans 75 ml de benzène et 15 ml de chlorure de thionyle.

On évapore sous vide, reprend avec 50 ml d'éther et coule dans un mélange sous agitation de 60 ml d'ammoniaque à 28 % et 100 g de glace. On agite 2 h, décante d'éther, lave à l'eau, sèche, évapore à sec sous vide, reprend le résidu à l'éther de pétrole et essore. On obtient l'amide attendu (F. = 73-74 °C) avec un rendement de 98 %.

3) CRL 40940.

On oxyde 13 g (0,046 mole) de 4,4'-difluorobenzhydrylthioacétamide en solution dans 40 ml d'acide acétique avec 4,6 ml de $H_2O_2$ à 110 volumes. On évapore sous vide, reprend à l'eau, essore, lave le

précipité à l'eau. Par recristallisation dans l'éthanol, on obtient le CRL 40940 avec un rendement global de 64 % (F. = 101-102 °C).

On a résumé ci-après les résultats des essais pharmacologiques qui ont été entrepris avec les produits selon la présente addition. Dans ces essais, les produits de formule I à étudier ont été administrés par voie intrapéritonéale, en suspension dans une solution aqueuse de gomme arabique, sous un volume de 20 ml/kg chez la souris mâle et sous un volume de 5 ml/kg chez le rat mâle.

I. Toxicité, comportement global et réactivités

La toxicité a été étudiée chez la souris mâle. Les résultats relatifs à la DL-O (dose minimale non mortelle) sont donnés dans le tableau II ci-après.

Pour apprécier le comportement global et les réactivités, des lots de six animaux (souris mâles ou rats mâles) ont été observés avant administration de chaque produit, puis 15 min, 30 min, 1 h, 2 h, 3 h et 24 h après administration de chaque produit. Les résultats sont consignés dans le tableau III ci-après.

Tableau II

Toxicité

| Produit | n° de code | DL-O |
|---|---|---|
| Exemple 1 | CRL 40920 | supérieure à 256 mg/kg |
| Exemple 2 | CRL 40929 | supérieure à 512 mg/kg |
| Exemple 4 | CRL 40936 | supérieure à 512 mg/kg |
| Exemple 5 | CRL 40489 | supérieure à 1024 mg/kg |
| Exemple 7 | CRL 40933 | supérieure à 256 mg/kg |
| Exemple 8 | CRL 40940 | supérieure à 256 mg/kg |
| Exemple 9 | CRL 41018 | supérieure à 512 mg/kg |

Tableau III

Comportement global et réactivités

| Produit (n° de code) | Observations |
|---|---|
| Exemple 1 (CRL 40920) | a) souris<br>à 256 mg/kg :<br>- dyspnée, excitation, démarche titubante, pas de mortalité<br>à 128 mg/kg :<br>- excitation<br>- hypothermie (-1,5°C) pendant 0,5 h<br>à 64 mg/kg :<br>- excitation<br><br>b) rat<br>à 64 mg/kg :<br>- mydriase pendant 3 h |

Tableau III (Suite)

| Produit (n° de code) | Observations |
|---|---|
| Exemple 2 (CRL 40929) | a) souris<br>à 512 mg/kg :<br>- respiration déprimée, dyspnée, sédation, diminution de la réactivité au toucher, puis excitation au bout de 3 h<br>- pas de mortalité<br>à 256 mg/kg :<br>- sédation pendant 0,5 h avec diminution de la fréquence respiratoire<br>- hyporéactivité au toucher pendant 2 h<br>- hypothermie modérée (-1°C) pendant 0,5 h<br>- excitation 3 h après administration<br>à 128 mg/kg :<br>- sédation, puis excitation 3 h après administration<br>b) rat<br>à 128 mg/kg :<br>- mydriase pendant 3 h |
| Exemple 7 (CRL 40933) | a) souris<br>à 1024 mg/kg :<br>- crampes abdominales, sédation et hyporéactivité au toucher, respiration déprimée<br>- mortalité tardive (66% des animaux) 24 h après administration<br>à 512 mg/kg :<br>- crampes abdominales, sédation et hyporéactivité au toucher<br>- mortalité tardive (33% des animaux) 24 h après administration |
| Exemple 7 | à 256 mg/kg :<br>- crampes abdominales, respiration déprimée, puis<br>- excitation apparaissant 1,5 h après administration<br>à 128 mg/kg :<br>- excitation apparaissant 1,5 h après |

Tableau III (Suite)

| Produit (n° de code) | Observations |
|---|---|
| (CRL 40933) | administration et durant 3 h<br>- réactivité au toucher<br>b) rat<br>à 64 mg/kg :<br>- hypotonie musculaire pendant 3 h<br>- respiration déprimée pendant 3 h<br>- mydriase pendant 2 à 3 h<br>à 16 mg/kg :<br>- mydriase 1 h après administration |
| Exemple 8<br>(CRL 40940)<br><br>Exemple 8<br>(CRL 40940) | a) souris<br>à 512 mg/kg :<br>- démarche titubante pendant 0,75 h,<br>- convulsions (66% des animaux) pendant 0,75 h<br>- mortalité (4 animaux sur 6 en 1 h, puis 2 animaux sur 6 en 24 h)<br>à 256 mg/kg :<br>- excitation débutant 0,5 h après administration<br>- stéréotypies<br>- pas de mortalité<br>à 128 mg/kg :<br>- excitation avec stéréotypies pendant 3 h<br>- augmentation de la réactivité au toucher pendant 24 h<br>- hypothermie (-1,3°C) pendant 3 h<br>à 32 mg/kg :<br>- excitation tardive débutant 1 h après administration et durant 2 h<br>- stéréotypies 3 h après administration<br>- réactivité au toucher pendant 24 h<br>à 8 mg/kg :<br>- excitation tardive débutant 2 h après administration<br>- réactivité au toucher pendant 2 h<br>à 2 mg/kg :<br>- réactivité au toucher pendant 2 h |

Tableau III (Suite)

| Produit (n° de code) | Observations |
|---|---|
| | b) rat<br>à 64 mg/kg :<br>- excitation<br>- réactivité au toucher<br>- fréquence respiratoire augmentée pendant 24 h<br>à 16 mg/kg :<br>- excitation<br>- réactivité au toucher pendant 2 h |
| Exemple 9<br>(CRL 41018) | a) souris<br>à 256 mg/kg :<br>- excitation apparaissant en 30 minutes et maximale 2 à 3 h après administration<br>- présence de stétréotypies nettes mais tardives<br>- réactivité au toucher pendant 3 h<br>à 64 mg/kg :<br>- excitation apparaissant en 30 minutes, maximale après 2 h et durant 24 h<br>- apparition tardive (2-3 h)- 4 mouvements stéréotypés de faible intensité<br>- réactivité au toucher pendant 2-3 h<br>à 16 mg/kg :<br>- excitation pendant 1 h pour 33 % des animaux<br>b) rat<br>à 128 mg/kg :<br>- excitation pendant 2 h avec hyper-réactivité au toucher pendant 0,5 h<br>à 32 mg/kg :<br>- diminution fugace (0,5 h) de la réactivité au toucher et du tonus musculaire<br>à 8 mg/kg :<br>- hyporéactivité fugace (0,5 h) et diminution du tonus musculaire |

II. Essais sur le SNC

Résultats des essais relatifs au CRL 40933 (produit de l'exemple 7).

A. Interaction avec l'apomorphine

1. Souris

Des lots de 6 souris par dose reçoivent le CRL 40933 0,5 h avant l'injection sous-cutanée d'apomorphine administrée à la dose de 1 ou 16 mg/kg.
On observe que, à la forte dose (128 mg/kg), le CRL 40933 s'oppose à l'action hypothermisante de 16 mg/kg d'apomorphine sans modifier le comportement de verticalisation et les stéréotypies.

2. Rat

Le CRL 40933 est administré à des lots de 6 rats 0,5 h avant l'injection sous-cutanée de 0,5 mg/kg d'apomorphine.
On constate que le CRL 40933 ne modifie pas le comportement stéréotypé induit par l'apomorphine chez le rat.

B. Interaction avec l'amphétamine

L'amphétamine (2 mg/kg) est injectée par voie intrapéritonéale à des lots de 6 rats, 30 min après l'administration de CRL 40933.
On remarque que le CRL 40933 ne modifie pas sensiblement les stéréotypies amphétaminiques ; les diminutions observées ne sont probablement dues qu'à l'existence d'un index de stéréotypies relativement fort qui est observé chez le lot témoin.

C. Interaction avec la réserpine

Quatre heures après l'injection intrapéritonéale de 2,5 mg/kg de réserpine, des lots de 6 souris reçoivent le CRL 40933.
Aux doses de 32 mg/kg et 128 mg/kg, on observe que le CRL 40933 s'oppose très faiblement à l'hypothermie réserpinique sans modifier le ptôsis.

D. Interaction avec l'oxotrémorine

Le CRL 40933 est administré à des lots de 6 souris 0,5 h avant l'injection intrapéritonéale de 0,5 mg/kg d'oxotrémorine. On observe ce qui suit :

1. Action sur la température

à forte dose (128 mg/kg) le CRL 40933 s'oppose faiblement à l'effet hypothermisant de l'oxotrémorine ;

2. Action sur les tremblements

les tremblements induits par l'oxotrémorine ne sont pas influencés par le CRL 40933 ;

3. Action sur les symptômes cholinergiques périphériques

le CRL 40933 ne modifie pas les signes de stimulation cholinergiques périphériques dus à l'oxotrémorine.

E. Action sur le test des quatre plaques, la traction et l'électrochoc

Les essais sont effectués sur des lots de 10 souris (par dose) 0,5 h après l'administration du CRL 40933.
On observe que le CRL 40933 n'entraîne pas d'augmentation nette du nombre de passages punis ; il ne provoque pas de déficit moteur majeur et, à dose élevée (32 mg/kg à 128 mg/kg), il s'oppose aux effets convulsivants de l'électrochoc.

F. Action sur la motilité spontanée

Une demi-heure après avoir reçu le CRL 40933, les souris (6 par dose, 12 témoins) sont placées en

actimètre où leur motilité est enregistrée pendant 30 min.

On constate que, aux doses de 32 mg/kg et 128 mg/kg, le CRL 40933 entraîne une augmentation nette de l'activité motrice spontanée de la souris. On note une diminution de motilité observée à la plus faible dose utilisée (2 mg/kg).

G. Action sur l'agressivité intergroupes

Après avoir séjourné pendant 3 semaines dans chacune des moitiés d'une cage séparées par une cloison opaque, des groupes de 3 souris reçoivent le CRL 40933. 30 min plus tard, les deux groupes d'une même cage sont réunis par retrait de la cloison et on note le nombre de combats qui surviennent en 10 min.

On observe que le CRL 40933 ne modifie pas nettement l'agressivité intergroupes.

H. Action vis-à-vis de quelques comportements perturbés par divers agents

1. Motilité réduite par habituation à l'enceinte

Après 18 h de séjour dans les actimètres, les souris (6 par dose, 12 témoins) reçoivent le CRL 40933. Elles sont aussitôt replacées dans leurs enceintes respectives et, 30 min plus tard, on enregistre leur motilité pendant 30 min.

On remarque que le CRL 40933 provoque une reprise de l'activité chez la souris habituée à son enceinte. Cet effet, à peine perceptible à 2 mg/kg et à 8 mg/kg est net à dose élevée (de 32 mg/kg à 128 mg/kg).

2. Motilité réduite par agression hypoxique

Une demi-heure après avoir reçu le CRL 40933, les souris (10 par dose, 20 témoins) sont soumises à une anoxie hypobare aiguë [dépression de 600 mm Hg (soit environ $8 \times 10^4$ pascals) en 90 s, puis détente de 45 s], puis elles sont placées en actimètre où leur motilité est enregistrée pendant 10 min.

On observe que, à forte dose (128 mg/kg), le CRL 40933 entraîne une amélioration nette de la récupération motrice chez les souris dont l'activité a été déprimée à la suite d'un séjour bref dans une enceinte à pression réduite.

3. Anoxie asphyxique

Des lots de 10 souris par dose reçoivent le CRL 40933 30 min. avant l'administration intrapéritonéale de 32 mg/kg de triiodoéthylate de gallamine.

A forte dose (128 mg/kg), le CRL 40933 diminue le délai d'apparition des convulsions et de la mort consécutives à une anoxie asphyxique provoquée par un curarisant.

I. Interaction avec le barbital

Une demi-heure après l'administration de CRL 40933, des lots de 10 souris reçoivent une injection intrapéritonéale de 220 mg/kg de barbital.

On observe que, dès la dose de 8 mg/kg, le CRL 40933 diminue la durée du sommeil induit par le barbital.

J. Action sur le « désespoir comportemental »

Une demi-heure après avoir reçu le CRL 40933, des lots de 10 souris par dose sont placés dans un espace restreint rempli d'eau sur une hauteur de 6 cm ; on note la durée d'immobilité entre la 2[e] et la 6[e] minute suivant l'immersion.

Le CRL 40933, aux doses de 8 mg/kg, 32 mg/kg, mais surtout 128 mg/kg, diminue la durée d'immobilité.

K. Conclusion

L'étude neuropsychopharmacologique du CRL 40933 met en évidence chez la souris mâle :

a) des effets de type stimulant :
- excitation et hyperréactivité,
- hypermotilité,
- reprise de l'activité motrice chez la souris habituée à son enceinte,
- amélioration de la récupération motrice après hypoxie hypobare,
- antagonisme du sommeil barbiturique ;

b) des effets de type antidépresseur qui sont faibles :
— antagonisme des hypothermies induites par l'apomorphine, l'oxotrémorine et la réserpine,
— diminution de l'immobilité « désespérée » ;

c) des effets anticonvulsivants :
— antagonisme des convulsions électriques.

Selon les modalités opératoires données ci-dessus, l'étude de l'action sur le SNC avec les autres produits a donné les résultats consignés ci-après.

Résultats des essais relatifs au CRL 40920 (produit de l'exemple 1).

A. Interaction avec l'apomorphine

1. Souris

A la dose de 128 mg/kg, le CRL 40920 est hypothermisant et ne s'oppose pas à l'hypothermie induite par l'apomorphine. Le CRL 40920 ne modifie pas les comportements de verticalisation et de stéréotypies.

2. Rat

Le CRL 40920 laisse inchangées les stéréotypies induites par l'apomorphine.

B. Interaction avec l'amphétamine

Le CRL 40920, aux doses utilisées, ne modifie pratiquement pas les stéréotypies amphétaminiques.

C. Interaction avec la réserpine

A la dose de 128 mg/kg, le CRL 40920 antagonise l'hypothermie induite par la réserpine sans modifier le ptôsis.

D. Interaction avec l'oxotrémorine

1. Action sur la température

Aux doses de 8 mg/kg à 128 mg/kg le CRL 40920 s'oppose modérément à l'action hypothermisante de l'oxotrémorine.

2. Action sur les tremblements

Le CRL 40920 ne modifie pas l'intensité des tremblements provoqués par l'oxotrémorine.

3. Action sur les symptômes cholinergiques périphériques

Le CRL 40920 laisse pratiquement inchangés les signes de stimulation cholinergique périphérique qui apparaissent après administration d'oxotrémorine.

E. Action sur le test des quatre plaques, la traction et l'électrochoc

Le CRL 40920 n'entraîne pas d'augmentation du nombre de passages punis ; il ne provoque pas d'incapacité motrice majeure et ne s'oppose pas aux effets convulsivants de l'électrochoc.

F. Action sur la motilité spontanée

Le CRL 40920, aux doses de 32 et 128 mg/kg, entraîne une augmentation modérée de la motilité spontanée de la souris.

G. Action sur l'agressivité intergroupes

Le CRL 40920 ne modifie pas nettement le nombre de combats.

H. Action vis-à-vis de quelques comportements perturbés par divers agents

1. Motilité réduite par habituation à l'enceinte

Aux doses de 8 mg/kg, 32 mg/kg et surtout 128 mg/kg, le CRL 40920 provoque une reprise d'activité motrice chez la souris habituée à son enceinte.

2. Motilité réduite par agression hypoxique

Le CRL 40920, à la dose de 128 mg/kg, entraîne une amélioration modérée (mais non significative) de la récupération motrice chez la souris dont la motilité a été déprimée à la suite d'un séjour bref dans une enceinte à pression réduite.

3. Anoxie asphyxique

Le CRL 40920 ne modifie pas le délai d'apparition des convulsions et de la mort consécutives à une anoxie asphyxique provoqués par un curarisant tel que le triiodoéthylate de gallamine.

I. Interaction avec le barbital

A la dose de 128 mg/kg, le CRL 40920 diminue nettement la durée du sommeil barbiturique. A dose plus faible (32 mg/kg), le CRL 40920 n'entraîne pas de modifications sensibles de la durée du sommeil mais une diminution nette du nombre de souris endormies.

J. Action sur le « désespoir comportemental »

Aux doses de 32 mg/kg et 128 mg/kg, le CRL 40920 diminue nettement la durée d'immobilité de la souris placée en immersion forcée.

K. Conclusion

L'étude neuropsychopharmacologique du CRL 40920 révèle essentiellement une composante excitante qui résulte de
— l'augmentation modérée de l'activité motrice ;
— la reprise de l'activité motrice chez la souris habituée à son enceinte ;
— l'antagonisme du sommeil barbiturique ;
— la diminution d'immobilité au test du désespoir comportemental.

De plus, il se pourrait que les antagonismes de certaines hypothermies ne soient que le reflet de la stimulation motrice.

Résultats des essais relatifs au CRL 40929 (produit de l'exemple 2).

A. Interaction avec l'apomorphine

1. Souris

Le CRL 40929 laisse inchangés l'hypothermie, le comportement de verticalisation et les stéréotypies produits par l'apomorphine chez la souris.

2. Rat

Les stéréotypies induites par l'apomorphine ne sont pas modifiées par le CRL 40929.

B. Interaction avec l'amphétamine

Le CRL 40929, aux doses de 8 mg/kg à 128 mg/kg, semble potentialiser faiblement les stéréotypies amphétaminiques.

C. Interaction avec la réserpine

A forte dose (256 mg/kg), le CRL 40929 diminue très modérément le ptôsis réserpinique.

D. Interaction avec l'oxotrémorine

1. Action sur la température

Le CRL 40929 ne modifie pratiquement pas l'action hypothermisante de l'oxotrémorine.

2. Action sur les tremblements

. A forte dose (256 mg/kg), le CRL 40929 semble diminuer modérément l'intensité des tremblements provoqués par l'oxotrémorine.

3. Action sur les symptômes cholinergiques périphériques

Aux doses élevées (de 64 mg/kg à 256 mg/kg), le CRL 40929 semble entraîner une diminution des signes de stimulation cholinergique périphérique, notamment la défécation.

E. Action sur le test des quatre plaques, la traction et l'électrochoc

Le CRL 40929 n'entraîne pas d'augmentation du nombre de passages punis ; il ne provoque pas de déficit moteur majeur mais, à forte dose (de 64 mg/kg à 256 mg/kg), s'oppose aux effets convulsivants de l'électrochoc.

F. Action sur la motilité spontanée

A la dose de 256 mg/kg, le CRL 40929 entraîne une hypermotilité nette.

G. Action sur l'agressivité intergroupes

On observe que le CRL 40929 est faiblement anti-agressif, car il diminue très modérément l'agressivité intergroupes chez la souris.

H. Action vis-à-vis de quelques comportements perturbés par divers agents

1. Motilité réduite par habituation à l'enceinte

Aux doses de 4 mg/kg, 64 mg/kg et 256 mg/kg, le CRL 40929 semble provoquer une reprise de l'activité chez la souris habituée à son enceinte. Un tel effet n'est pas observé à la dose intermédiaire de 16 mg/kg.

2. Motilité réduite par agression hypoxique

Aux doses de 64 mg/kg et surtout 256 mg/kg, le CRL 40929 entraîne une amélioration nette de la récupération motrice chez la souris habituée à son enceinte.

3. Anoxie asphyxique

Aux doses de 64 mg/kg et 256 mg/kg, le CRL 40929 retarde modérément l'apparition des convulsions anoxiques provoquées par le triiodoéthylate de gallamine.

I. Interaction avec le barbital

A la forte dose (256 mg/kg), le CRL 40929 diminue nettement la durée du sommeil barbiturique.

J. Action sur le « désespoir comportemental »

On observe que, dès la dose de 16 mg/kg, le CRL 40929 diminue nettement la durée d'immobilité.

K. Conclusion

L'étude neuropsychopharmacologique met en évidence que le CRL 40929 est un agent psychotrope du fait de :
— son action stimulante de la motilité spontanée, de la motilité réduite par habituation,
— son amélioration de la récupération motrice après agression hypoxique,
— son effet dans le test dit du « désespoir comportemental ».

Résultats des essais relatifs au CRL 40936 (produit de l'exemple 4)

Le CRL 40936 présente aux doses faibles et moyennes chez la souris un ensemble d'effets de type sédatif (sédation, hyporéactivité, hypoagressivité, hypothermie) ainsi qu'un antagonisme de l'effet convulsivant de l'électrochoc. Ces effets sont observés cependant en l'absence de toute diminution d'activité motrice.

En revanche, aux doses élevées, le CRL 40936 provoque l'apparition d'une hypermotilité discrète, d'une reprise modérée de l'activité chez la souris habituée à son enceinte et une amélioration sensible de la récupération motrice après hypoxie.

De plus, un effet antagoniste du sommeil barbiturique et une diminution de l'hypomotilité de « désespoir » sont observés.

Résultats des essais relatifs au CRL 40489 (produit de l'exemple 5)

Le CRL 40489 se présente comme un agent actif sur le SNC, de type sédatif. Au cours du test relatif à la motilité spontanée, on observe que le CRL 40489 provoque une hypomotilité à toutes les doses utilisées (8 mg/kg à 256 mg/kg), l'intensité de cette hypomotilité croissant avec la dose. En revanche, le CRL 40489 n'agit pas nettement sur la mobilité réduite (habituation à l'enceinte et agression hypoxique). Le CRL 40489 présente en outre un effet antiagressif. A la dose de 32 mg/kg, il diminue de moitié l'agressivité intergroupes.

Résultats des essais relatifs au CRL 40940 (produit de l'exemple 8)

A. Interaction avec l'apomorphine

1. Souris

Aux doses de 32 mg/kg et 128 mg/kg, le CRL 40940 est hypothermisant et aggrave l'hypothermie induite par les doses de 1 et 16 mg/kg d'apomorphine, en revanche, il ne modifie pas le comportement de verticalisation et les stéréotypies.

2. Rat

A la dose de 64 mg/kg, le CRL 40940 augmente les stéréotypies induites par l'apomorphine.

B. Interaction avec l'amphétamine

A la dose de 64 mg/kg, le CRL 40940 entraîne une potentialisation des stéréotypies amphétaminiques.

C. Interaction avec la réserpine

Aux doses de 32 mg/kg, 64 mg/kg et 128 mg/kg, le CRL 40940 antagonise tardivement l'hypothermie réserpinique ; il antagonise également mais de façon modérée le ptôsis réserpinique à 24 h.

D. Interaction avec l'oxotrémorine

1. Action sur la température

Aux doses de 2 mg/kg, 8 mg/kg, 32 mg/kg et 128 mg/kg, le CRL 40940 antagonise modérément et tardivement l'hypothermie induite par l'oxotrémorine.

2. Action sur les tremblements

Les tremblements dus à l'oxotrémorine ne sont pratiquement pas modifiés.

3. Action sur les symptômes cholinergiques périphériques

Le CRL 40940 ne modifie pas les signes de stimulation cholinergique périphérique dus à l'oxotrémorine.

E. Action sur le test des quatre plaques, la traction et l'électrochoc

Le CRL 40940 n'entraîne pas d'augmentation du nombre de passages punis ; il ne provoque pas d'incapacité motrice majeure et ne modifie pratiquement pas les effets convulsivants de l'électrochoc.

F. Action sur la motilité spontanée

Le CRL 40940, aux doses de 8 mg/kg, 32 mg/kg et 128 mg/kg, entraîne une augmentation nette de la motilité spontanée de la souris.

G. Action sur l'agressivité intergroupes

Les essais effectués montrent que le CRL 40940 n'a pas d'effet antiagressif.

H. Action vis-à-vis de quelques comportements perturbés par divers agents

1. Motilité réduite par habituation à l'enceinte

Dès la dose de 8 mg/kg et surtout à 32 mg/kg et 128 mg/kg, le CRL 40940 provoque une reprise d'activité motrice très nette chez la souris habituée à son enceinte.

2. Motilité réduite par agression hypoxique

Le CRL 40940, aux doses de 32 mg/kg, mais surtout 128 mg/kg, entraîne une amélioration significative de la récupération motrice chez la souris dont la motilité a été déprimée à la suite d'un séjour bref dans une enceinte à pression réduite.

3. Anoxie asphyxique

Le CRL 40940 ne modifie pas le délai d'apparition des convulsions et de la mort consécutives à une anoxie asphyxique provoquée par un curarisant tel que le triiodoéthylate de gallamine.

I. Interaction avec le barbital

Aux doses de 32 mg/kg et 128 mg/kg, le CRL 40940 diminue nettement la durée du sommeil barbiturique.

J. Action sur le « désespoir comportemental »

Aux doses de 8 mg/kg, 32 mg/kg et 128 mg/kg, le CRL 40940 diminue nettement la durée d'immobilité de la souris placée en immersion forcée.

K. Conclusion

L'étude neuropsychopharmacologique du CRL 40940 met essentiellement en évidence une composante excitante qui résulte de :
— l'excitation avec hyperréactivité,
— l'augmentation de l'activité motrice,
— la reprise de l'activité motrice chez la souris habituée à son enceinte,
— l'antagonisme du sommeil barbiturique,
— la diminution d'immobilité au test du « désespoir comportemental », et
— l'amélioration de la récupération motrice après anoxie asphyxique.

Ces effets, à la différence des stimulants amphétaminiques, apparaissent en l'absence de toxicité particulière chez les souris groupées, et de signes de stimulation sympathique périphérique.

L'apparition et le développement relativement tardif d'un certain nombre d'effets (hyperréactivité, antagonisme de l'hypothermie et du ptôsis réserpiniques, antagonisme de l'hypothermie induite par l'oxotrémorine) laissent supposer une résorption lente du CRL 40940. Cette supposition semble confirmée par l'étude cinétique des stéréotypies.

Résultats des essais relatifs au CRL 41018 (produit de l'exemple 9)

A. Interaction avec l'apomorphine

Chez la souris, à la dose de 256 mg/kg, le CRL 41018 s'oppose à l'action hypothermisante de l'apomorphine sans modifier le comportement de verticalisation et les stéréotypies.

B. Interaction avec la réserpine

A la dose de 256 mg/kg on observe que le CRL 41018 s'oppose tardivement à l'hypothermie (1,5 h) et au ptôsis (24 h) réserpiniques.

C. Interaction avec l'oxotrémorine

1. Action sur la température

A la dose la plus forte utilisée, le CRL 41018 s'oppose à la baisse de température provoquée par l'oxotrémorine.

2. Action sur les tremblements

L'intensité des tremblements provoqués par l'oxotrémorine n'est pratiquement pas modifiée par le CRL 41018.

3. Action sur les symptômes cholinergiques périphériques

Le CRL 41018 ne modifie pas les signes de stimulation cholinergique périphérique induits par l'oxotrémorine.

D. Action sur le test des quatre plaques, la traction et l'électrochoc

Aux doses de 64 et 256 mg/kg, le CRL 41018 entraîne une nette augmentation des passages punis ; il ne provoque pas de déficit moteur ; et à forte dose (256 mg/kg) il s'oppose partiellement aux effets convulsivants de l'électrochoc.

E. Action sur la motilité spontanée

On observe que le CRL 41018 provoque dès la dose de 16 mg/kg une hypermotilité ; on constate par ailleurs que l'hypermotilité est maximale 0,5 h après administration et que la durée de l'hypermotilité est au moins de 4 h.

F. Motilité réduite par habituation à l'enceinte

On constate que aux doses de 64 mg/kg et 256 mg/kg, le CRL 41018 provoque une reprise de l'activité motrice chez la souris habituée à son enceinte.

G. Interaction avec le barbital

Aux doses de 16 mg/kg, 64 mg/kg et 256 mg/kg, le CRL 41018 diminue nettement la durée du sommeil barbiturique.

H. Action sur le « désespoir comportemental »

On observe que le CRL 41018 aux doses de 16 mg/kg, 64 mg/kg et 256 mg/kg diminue fortement la durée d'immobilité.

I. Conclusion

L'étude neuropsychopharmacologique du CRL 41018 met en évidence un effet stimulant du SNC qui est essentiel, et à forte dose seulement un effet antidépresseur du SNC.

III. Essais cliniques

Les études cliniques ont porté sur les troubles de la vigilance, de l'éveil et des états confusionnels, d'une part, et sur les troubles de l'affectivité, du contact et des dépressions, d'autre part.

Chez l'homme, notamment chez le vieillard, on a observé que les CRL 40929, CRL 40933, CRL 40940 et CRL 41018 administrés sous forme de gélules ou de comprimés (renfermant chacun de 100 à 200 mg d'ingrédient actif à raison de 1 à 3 gélules ou comprimés par jour), ont donné d'excellents résultats en tant que médicaments de l'éveil. On a également constaté que ces quatre produits agissent efficacement contre l'énurésie.

**Revendications**

(pour les Etats contractants : BE, CH, DE, FR, GB, IT, LI, LU, NL, SE)

1. Nouveau dérivé de benzhydrylsulfinylacétamide, caractérisé en ce qu'il est choisi parmi l'ensemble constitué par les composés répondant à la formule générale :

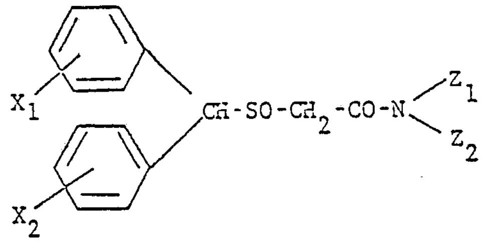

dans laquelle :

— $X_1$ et $X_2$, identiques ou différents, représentent chacun H, Cl ou F,

— $Z_1$ représente $CH_3$, $CH_2CH_3$, $CH(CH_3)_2$ ou $C(CH_3)_3$, $Z_1$ pouvant représenter l'atome d'hydrogène quand au moins un des $X_1$ et $X_2$ est différent de H,

— $Z_2$ représente H ; $NZ_1Z_2$ considérés ensemble pouvant représenter un groupe pipéridino ou morpholino.

2. Nouveau dérivé de benzhydrylsulfinylacétamide selon la revendication 1, caractérisé en ce que $X_1$ et $X_2$, qui peuvent être identiques ou différents, représentent chacun H, 4-Cl ou 4-F.

3. Nouveau dérivé de benzhydrylsulfinylacétamide selon la revendication 1, caractérisé en ce qu'il est choisi parmi l'ensemble constitué par les :

N-méthyl-benzhydrylsulfinylacétamide,
N-éthyl-benzhydrylsulfinylacétamide,
N-isopropyl-benzhydrylsulfinylacétamide,
N-tertiobutyl-benzhydrylsulfinylacétamide,
4-chloro-benzhydrylsulfinylacétamide,
4,4'-difluoro-benzhydrylsulfinylacétamide,
N-(benzhydrylsulfinylacétyl)-pipéridine,
N-(benzhydrylsulfinylacétyl)-morpholine, et
4-fluoro-benzhydrylsulfinylacétamide.

4. N-Isopropyl-benzhydrylsulfinylacétamide.
5. 4-Chloro-benzhydrylsulfinylacétamide.
6. 4,4'-Difluoro-benzhydrylsulfinylacétamide.
7. N-(Benzhydrylsulfinylacétyl)-pipéridine.
8. 4-Fluoro-benzhydrylsulfinylacétamide.
9. N-Ethyl-benzhydrylsulfinylacétamide.

10. Composition thérapeutique, caractérisée en ce qu'elle renferme, en association avec un excipient physiologiquement acceptable, au moins un dérivé de benzhydrylsulfinylacétamide selon l'une quelconque des revendications 1 à 9.

**Revendications** (pour l'Etat contractant AT)

1. Procédé de préparation d'un nouveau dérivé de benzhydrylsulfinylacétamide répondant à la formule générale :

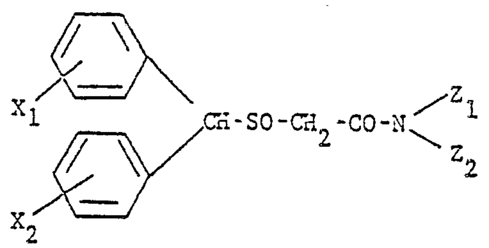

(I)

dans laquelle :

— $X_1$ et $X_2$ identiques ou différents, représentent chacun H, Cl ou F,

— $Z_1$ représente $CH_3$, $CH_2CH_3$, $CH(CH_3)_2$ ou $C(CH_3)_3$, $Z_1$ pouvant représenter l'atome d'hydrogène quand au moins un des $X_1$ et $X_2$ est différent de H,

— $Z_2$ représente H ; $NZ_1Z_2$ considérés ensemble pouvant représenter un groupe pipéridino ou morpholino ;

ledit procédé étant caractérisé en ce que, on fait réagir un chlorure d'acide de formule

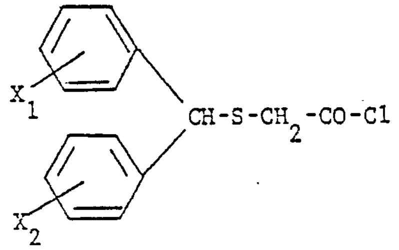

(II)

(où $X_1$ et $X_2$ sont définis comme ci-dessus) avec une amine

$HNZ_1Z_2$ (III)

(où $Z_1$ et $Z_2$ sont définis comme ci-dessus) pour obtenir un dérivé de benzhydrylthioacétamide de formule

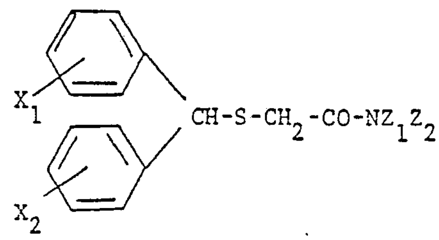

(IV)

(où $X_1$, $X_2$, $Z_1$ et $Z_2$ sont définis comme ci-dessus), et on soumet ledit dérivé de benzhydrylthioacétamide à une réaction d'oxydation au moyen de $H_2O_2$ à 110 130 volumes à une température inférieure ou égale à 45 °C.

2. Procédé selon la revendication 1 caractérisé en ce que $X_1$ et $X_2$, identiques ou différents, sont choisis parmi l'hydrogène, ou le chlore, ou le fluor, ces derniers étant en position 4.

3. Procédé selon l'une des revendications 1 à 2 caractérisé en ce que le chlorure d'acide de formule (II) dans laquelle $X_1$ et $X_2$ sont H, est mis en réaction avec l'isopropylamine.

4. Procédé selon l'une des revendications 1 et 2 caractérisé en ce que le chlorure d'acide de formule (II) dans laquelle $X_1$ est H, et $X_2$ est 4-Cl, est mis en réaction avec l'ammoniac.

5. Procédé selon l'une des revendications 1 et 2 caractérisé en ce que le chlorure d'acide de formule (II) dans laquelle $X_1$ et $X_2$ représentent 4-F, est mis en réaction avec l'ammoniac.

6. Procédé selon l'une des revendications 1 et 2 caractérisé en ce que le chlorure d'acide de formule (II) dans laquelle $X_1$ et $X_2$ représentent H, est mis en réaction avec la pipéridine.

7. Procédé de préparation selon l'une des revendications 1 et 2 caractérisé en ce que le chlorure d'acide de formule (II) dans laquelle $X_1$ représente H, et $X_2$ représente 4-F, est mis en réaction avec l'ammoniac.

8. Procédé de préparation selon l'une des revendications (1) et (2) caractérisé en ce que le chlorure d'acide de formule (II) dans laquelle $X_1$ et $X_2$ représentent H est mis en réaction avec l'éthylamine.

9. Utilisation d'un produit de formule (I) que l'on peut obtenir par le procédé décrit dans l'une des revendications 1 à 8 à la préparation de compositions thérapeutiques actives sur le système nerveux central, lesdites compositions contenant au moins un produit de formule (I) en association avec un excipient physiologiquement acceptable.

**Claims** (for the Contracting States : BE, CH, DE, FR, GB, IT, LI, LU, NL, SE)

1. A novel benzhydrylsulfinylacetamide derivative characterized in that it is selected from the group consisting of compounds of the general formula :

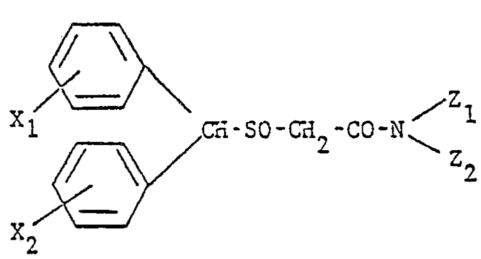

(I)

wherein

— $X_1$ and $X_2$, which may be identical or different each represent H, Cl or F,

— $Z_1$ represents $CH_3$, $CH_2CH_3$, $CH(CH_3)_2$ or $C(CH_3)_3$, $Z_1$ may represent the hydrogen atom when at least one of $X_1$ and $X_2$ is different from H,

— $Z_2$ represents H ; $NZ_1Z_2$ considered together may represent a piperidino or morpholino group.

2. A novel benzhydrylsulfinylacetamide derivative according to formula I according to claim 1, characterized in that $X_1$ and $X_2$, which may be identical or different, each represent H, 4-Cl or 4-F.

3. A novel benzhydrylsulfinylacetamide derivative according to claim 1, characterized in that it is selected from the group consisting of :

N-methyl-benzhydrylsulfinylacetamide,
N-ethyl-benzhydrylsulfinylacetamide,

N-isopropyl-benzhydrylsulfinylacetamide,
N-tertiobutyl-benzhydrylsulfinylacetamide,
4-chloro-benzhydrylsulfinylacetamide,
4,4'-difluoro-benzhydrylsulfinylacetamide,
N-(benzhydrylsulfinylacetyl)-piperidine,
N-(benzhydrylsulfinylacetyl)morpholine and
4-fluoro-benzhydrylsulfinylacetamide.

4. N-Isopropyl-benzhydrylsulfinylacetamide.
5. 4-Chloro-benzhydrylsulfinylacetamide.
6. 4.4'-Difluoro-benzhydrylsulfinylacetamide.
7. N-(Benzhydrylsulfinylacetyl)-piperidine.
8. 4-Fluoro-benzhydrylsulfinylacetamide.
9. N-Ethyl-benzhydrylsulfinylacetamide.
10. A therapeutical composition, characterized in that it comprises in association with a physiologically acceptable excipient, at least a benzhydrylsulfinylacetamide derivative according to any one of claims 1 to 9.

**Claims** (for the contracting state AT)

1. A method for the preparation of a novel benzhydrylsulfinylacetamide derivative of the general formula :

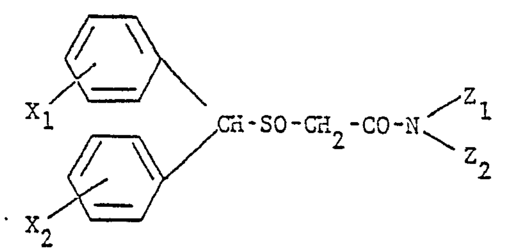

(I)

wherein :

— $X_1$ and $X_2$, which may be identical or different each represent H, Cl or F,

— $Z_1$ represents $CH_3$, $CH_2CH_3$, $CH(CH_3)_2$ or $C(CH_3)_3$, $Z_1$ may represent the hydrogen atom when at least one of $X_1$ and $X_2$ is different from H,

— $Z_2$ represents H ; $NZ_1Z_2$ considered together may represent a piperidino or morpholino group, characterised in that an acid chloride of general formula

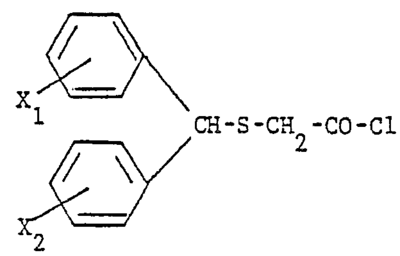

(II)

wherein $X_1$ and $X_2$ are as defined above, is reacted with an amine of general formula

$$HNZ_1Z_2 \quad (III)$$

wherein $Z_1$ and $Z_2$ have the above definition, to a benzhydrylthioacetamide derivative of general formula

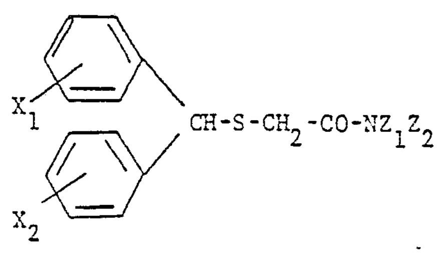

(IV)

wherein $X_1$, $X_2$, $Z_1$ and $Z_2$ are as defined above, and the obtained benzhydrylthioacetamide derivative of formula (IV) is oxidised.

2. A method according to claim 1, characterised in that the derivative of formula (IV) is oxidised with $H_2O_2$ of a concentration of 110 to 130 parts of volume of oxygen at a temperature below or equal to 45 °C.

3. A method according to claim 1, characterised in that an acid chloride of formula (II) is used wherein $X_1$ and $X_2$ are identical or different and each represent hydrogen or chloride or fluorine in position 4.

4. A method according to claims 1 to 3, for the preparation of N-isopropyl-benzhydrylsulfinylacetamide, characterised in that an acid chloride of formula (II), wherein $X_1$ and $X_2$ represent hydrogen, is reacted with isopropyl-amine.

5. A method according to claims 1 to 3, for the preparation of 4-chloro-benzhydrylsulfinylacetamide, characterised in that an acid chloride of formula (II), wherein $X_1$ represents hydrogen and $X_2$ 4-Cl, is reacted with ammonia.

6. A method according to claims 1 to 3, for the preparation of 4,4'-difluoro-benzhydrylsulfinylacetamide, characterised in that an acid chloride of formula (II), wherein $X_1$ and $X_2$ represent 4-F, is reacted with ammonia.

7. A method according to claims 1 to 3, for the preparation of N-(benzhydrylsulfinylacetyl)-piperidine, characterised in that an acid chloride of formula (II), wherein $X_1$ and $X_2$ represent hydrogen is reacted with piperidine.

8. A method according to claims 1 to 3, for the preparation of 4-fluoro-benzhydrylsulfinylacetamide, characterised in that an acid chloride of formula (II), wherein $X_1$ represents hydrogen an $X_2$ 4-F, is reacted with ammonia.

9. A method according to claims 1 to 3, for the preparation of N-ethyl-benzhydrylsulfinylacetamide, characterised in that an acid chloride of formula (II), wherein $X_1$ and $X_2$ represent hydrogen, is reacted with ethylamine.

10. Application of the novel benzhydrylsulfinylacetamide derivatives of formula (I) obtainable according to claims 1 to 9, for the preparation of therapeutical compositions acting on the central nervous system, which contain at least a derivative of general formula (I) as active principle in association with a physiologically acceptable excipient.

**Patentansprüche** (für die Vertragsstaaten : BE, CH, DE, FR, GB, IT, LI, LU, NL, SE)

1. Neues Benzhydrylsulfinylacetamid-Derivat, dadurch gekennzeichnet, daß es ausgewählt ist aus der Gruppe bestehend aus den Verbindungen der allgemeinen Formel :

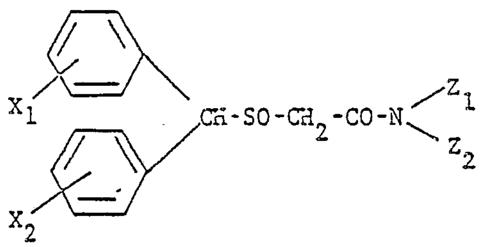

(I)

worin :

— $X_1$ und $X_2$, gleich oder verschieden, jeweils für H, Cl oder F stehen,

— $Z_1$ $CH_3$, $CH_2CH_3$, $CH(CH_3)_2$ oder $C(CH_3)_3$ darstellt, wobei $Z_1$ ein Wasserstoffatom sein kann, wenn wenigstens eines von $X_1$ und $X_2$ ungleich H ist,

— $Z_2$ für H steht ; wobei $NZ_1Z_2$, zusammen betrachtet, eine Piperidino- oder Morpholinogruppe bedeuten können.

2. Neues Benzhydrylsulfinylacetamid-Derivat nach Anspruch 1, dadurch gekennzeichnet, daß $X_1$ und $X_2$, die gleich oder verschieden sein können, jeweils für H, 4-Cl oder 4-F stehen.

3. Neues Benzhydrylsulfinylacetamid-Derivat nach Anspruch 1, dadurch gekennzeichnet, daß es ausgewählt ist aus der Gruppe bestehend aus :

N-Methyl-benzhydrylsulfinylacetamid,
N-Äthyl-benzhydrylsulfinylacetamid,
N-Isopropyl-benzhydrylsulfinylacetamid,
N-tert.Butyl-benzhydrylsulfinylacetamid,
4-Chlor-benzhydrylsulfinylacetamid,
4,4'-Difluor-benzhydrylsulfinylacetamid,
N-(Benzhydrylsulfinylacetyl)-piperidin,
N-(Benzhydrylsulfinylacetyl)-morpholin und
4-Fluor-benzhydrylsulfinylacetamid.

4. N-Isopropyl-benzhydrylsulfinylacetamid.
5. 4-Chlor-benzhydrylsulfinylacetamid.
6. 4,4'-Difluor-benzhydrylsulfinylacetamid.
7. N-(Benzhydrylsulfinylacetyl)-piperidin.
8. 4-Fluor-benzhydrylsulfinylacetamid.
9. N-Äthyl-benzhydrylsulfinylacetamid.
10. Therapeutische Zusammensetzung, dadurch gekennzeichnet, daß sie in Verbindung mit einem physiologisch akzeptablen Exzipienten mindestens ein Benzhydrylsulfinylacetamid-Derivat nach einem der Ansprüche 1 bis 9 enthält.

**Patentansprüche** (für den Vertragsstaat AT)

1. Verfahren zur Herstellung von neuen Benzhydrylsulfinylacetamid-Derivaten der allgemeinen Formel :

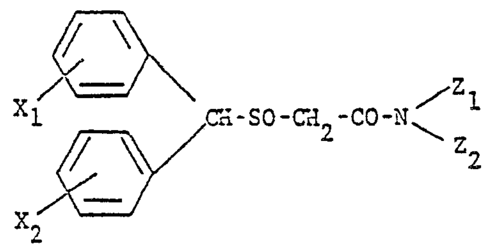

(I)

worin :

— $X_1$ und $X_2$, gleich oder verschieden, jeweils für H, Cl oder F stehen,

— $Z_1$ $CH_3$, $CH_2CH_3$, $CH(CH_3)_2$ oder $C(CH_3)_3$ darstellt, wobei $Z_1$ ein Wasserstoffatom sein kann, wenn wenigstens eines von $X_1$ und $X_2$ ungleich H ist,

— $Z_2$ für H steht ; wobei $NZ_1Z_2$, zusammen betrachtet, eine Piperidino- oder Morpholinogruppe bedeuten können, dadurch gekennzeichnet, daß ein Säurechlorid der allgemeinen Formel

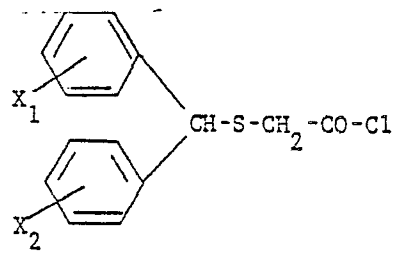

(II)

worin $X_1$ und $X_2$ wie voranstehend definiert sind, mit einem Amin der allgemeinen Formel

$$HNZ_1Z_2 \qquad (III)$$

worin $Z_1$ und $Z_2$ obige Bedeutung haben, zu einem Benzhydrylthioacetamid-Derivat der allgemeinen Formel

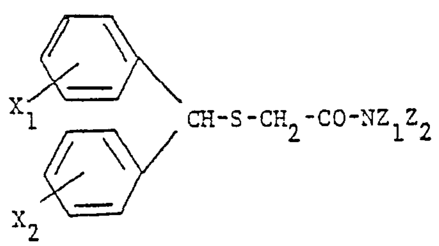

(IV)

worin $X_1$, $X_2$, $Z_1$ und $Z_2$ wie oben definiert sind, umgesetzt wird und das erhaltene Benzhydrylthioacetamid-Derivat der Formel (IV) oxidiert wird.

2. Verfahren nach Anspruch 1, dadurch gekennzeichnet, daß das Derivat der Formel (IV) mit $H_2O_2$ einer Konzentration von 110 bis 130 Volumenteilen Sauerstoff bei einer Temperatur unter oder gleich 45 °C oxidiert wird.

3. Verfahren nach Anspruch 1, dadurch gekennzeichnet, daß ein Säurechlorid der Formel (II)

eingesetzt wird, worin $X_1$ und $X_2$ gleich oder verschieden sind und jeweils für Wasserstoff oder Chlor oder Fluor in Position 4 stehen.

4. Verfahren nach den Ansprüchen 1 bis 3 zur Herstellung von N-Isopropyl-benzhydrylsulfinylacetamid, dadurch gekennzeichnet, daß ein Säurechlorid der Formel (II), worin $X_1$ und $X_2$ Wasserstoff bedeuten, mit Isopropylamin umgesetzt wird.

5. Verfahren nach den Ansprüchen 1 bis 3, zur Herstellung von 4-Chlor-benzhydrylsulfinylacetamid, dadurch gekennzeichnet, daß ein Säurechlorid der Formel (II) worin $X_1$ Wasserstoff und $X_2$ 4-Cl bedeuten, mit Ammoniak umgesetzt wird.

6. Verfahren nach den Ansprüchen 1 bis 3 zur Herstellung von 4,4'-Difluor-benzhydrylsulfinylacetamid, dadurch gekennzeichnet, daß ein Säurechlorid der Formel (II), worin $X_1$ und $X_2$ für 4-F stehen, mit Ammoniak umgesetzt wird.

7. Verfahren nach den Ansprüchen 1 bis 3 zur Herstellung von N-(Benzhydrylsulfinylacetyl)-piperidin, dadurch gekennzeichnet, daß ein Säurechlorid der Formel (II), worin $X_1$ und $X_2$ Wasserstoff bedeuten, mit Piperidin umgesetzt wird.

8. Verfahren nach den Ansprüchen 1 bis 3 zur Herstellung von 4-Fluor-benzhydrylsulfinylacetamid, dadurch gekennzeichnet, daß ein Säurechlorid der Formel (II), worin $X_1$ Wasserstoff und $X_2$ 4-F darstellen, mit Ammoniak umgesetzt wird.

9. Verfahren nach den Ansprüchen 1 bis 3 zur Herstellung von N-Äthyl-benzhydrylsulfinylacetamid, dadurch gekennzeichnet, daß ein Säurechlorid der Formel (II), worin $X_1$ und $X_2$ für Wasserstoff stehen, mit Äthylamin umgesetzt wird.

10. Verwendung der neuen, nach den Ansprüchen 1 bis 9 erhältlichen Benzhydrylsulfinylacetamid-Derivate der Formel (I) zur Herstellung von auf das Zentralnervensystem wirkenden therapeutischen Zusammensetzungen, welche mindestens ein Derivat der allgemeinen Formel (I) als Wirkstoff zusammen mit einem physiologisch unbedenklichen Exzipienten enthalten.